(19) Europäisches Patentamt
European Patent Office
Office européen des brevets

(11) Publication number: **0 438 873 A1**

(12) **EUROPEAN PATENT APPLICATION**

(21) Application number: **90313148.0**

(51) Int. Cl.$^5$: **C07K 7/06,** A61K 37/64

(22) Date of filing: **04.12.90**

The title of the invention has been amended (Guidelines for Examination in the EPO, A-III, 7.3).

(30) Priority: **07.12.89 US 447357**

(43) Date of publication of application: **31.07.91 Bulletin 91/31**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900 (US)**

(72) Inventor: **Ashton, Wallace T.**
**122 Sweet Briar Drive**
**Clark, NJ 07066 (US)**
Inventor: **Chang, Linda L.**
**45 Brandon Avenue**
**Wayne, NJ 07470 (US)**
Inventor: **Hannah, John**
**155 Idlebrook Lane**
**Matawan, NJ 07747 (US)**
Inventor: **Rasmusson, Gary H.**
**155 Park Place**
**Watchung, NJ 07060 (US)**
Inventor: **Tolman, Richard L.**
**29 Upper Warren Way**
**Warren, NJ 07060 (US)**

(74) Representative: **Hesketh, Alan, Dr. et al**
**European Patent Department Merck & Co., Inc. Terlings Park Eastwick Road**
**Harlow Essex, CM20 2QR (GB)**

(54) **Inhibitor of ribonucleotide reductase of herpes viruses.**

(57) A series of substituted peptides have been found to possess antiviral potency - specifically against herpes viruses - by selectively inhibiting the viral ribonucleotide reductase enzyme.

EP 0 438 873 A1

# PEPTIDE ANTIVIRAL AGENTS

## BACKGROUND OF THE INVENTION

Herpes simplex viruses (HSV) cause a wide spectrum of diseases ranging from mild to severe mucosal lesions, keratitis, and encephalitis. Both HSV types 1 and 2 are widely distributed in the western adult population, with reported exposure rates to HSV-1 estimated to be as high as 90%. The recent and rapid increase in the number of genital HSV infections is reflected by serology studies which indicate that 15-35% of the North American adult population have been exposed to HSV-2.

The major effort to develop antiherpetic drugs has historically centered on nucleoside analog inhibitors of HSV DNA polymerase. All currently used therapies are nucleoside analogs, acyclovir being the prime example. Oral or IV acyclovir is the therapy of choice for most infections. Topical acyclovir, vidarabine or idoxuridine are all used for herpes keratitis, the leading cause of corneal blindness in this country. However, considering the complex replication cycle and large number of virally encoded proteins, other potential targets for antiviral drugs must exist. HSV ribonucleotide reductase (RR) is one such target ; the viral-specified enzyme is markedly different from mammalian counterparts. HSV-RR catalyzes the reduction of the four ribonucleotides to the corresponding deoxyribonucleotides required for DNA replication. Published analysis of viral RR mutants indicated that the enzyme is not essential for growth of herpes in culture (Goldstein and Weller, Virology 166 : 41 (1988)), but is essential in vivo (Spector, Pharmacol. Ther., 31, 295 (1985)). Herpes RR inhibitors have been shown to possess antiherpetic activity per se (Shipman et al., Antiviral Research, 6 : 197 (1986)) and also to potentiate or synergize the action of acyclonucleoside antiviral agents (Spector et al., Proc. of the Nat. Acad. of Sci., 86: 1051 (1989)).

Dutia et al., Nature 321 : 439-441 (1986) and Cohen et al., Nature 321 : 441-443 (1986) and U.S. Patent No. 4,795,740, both disclosed that the nonapeptide Tyr Ala Gly Ala Val Val Asn Asp Leu, inhibited in vitro the activity of this enzyme. In addition, Dutia et al., op. cit., also disclosed that its 8-desalanine homolog, Tyr Gly Ala Val Val Asn Asp Leu, also inhibited in vitro the activity of this enzyme. Gaudreau et al., J. Biol. Chemistry, 262 : 12413, (1987) disclosed structure-activity studies of analogs of the nonapeptide described above. Garsky and Stein, U.S. Patent No. 4,837,304, disclose a series of oligopeptides that have been shown to inhibit in vitro the activity of the RR enzyme.

## OBJECTS OF THE INVENTION

It is the object of the present invention to provide novel substituted peptides which inhibit the activity of the ribonucleotide reductase enzyme of viruses, particularly viruses of the herpes family of viruses and especially the herpes simplex virus. Another object is to provide inhibitor peptides that lack or show weak activity against mammalian ribonucleotide reductase.

## SUMMARY OF THE INVENTION

A series of substituted peptides have been found to inhibit the activity of the ribonucleotide reductase enzyme of herpes simplex virus in vitro.

The present invention provides novel substituted peptide compounds of the Formula I :

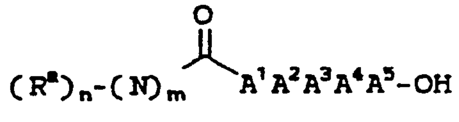

I

wherein :

$A^1$ and $A^2$ are independently :

a) isoleucine ;
b) leucine ;
c) norleucine ;
d) valine ;
e) cyclohexylglycine ;
f) phenylglycine ;

g) N-methyl isoleucine ;
h) N-methyl valine ;
i) phenylalanine ;
j) N-methyl leucine ;

or any of the enantiomorphic forms thereof ;

$A^3$ is

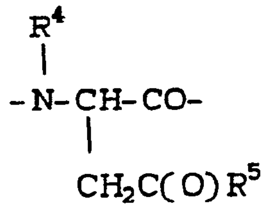

wherein :

$R^4$ is hydrogen or methyl ;
$R^5$ is $-OR^8$ or $NR^6R^7$
where $R^6$ and $R^7$ are independently :
j) hydrogen ;
k) $C_3$-$C_7$ cycloalkyl ;
l) $C_5$-$C_7$ cycloalkenyl ;
m) phenyl ;
n) phenyl substituted by : $-NH_2$.
$C_1$-$C_4$alkyl, $-SR^8$, $-CO_2H$ or $OR^8$ ;
where $R^8$ is H or $C_1$-$C_4$ alkyl ;
o) polycyclic aromatic ;
p) $C_1$-$C_8$ alkyl ;
q) $C_1$-$C_4$ alkyl monosubstituted by k)-n) hereinabove.

or $R^6$ and $R^7$ are combined to form a $C_3$-$C_5$ diradical or $-NR^6R^7$ is morpholino ;

$A_4$ is aspartic acid, N-methyl aspartic acid, or any of the enantiomorphic forms thereof ;

$A^5$ is an amino acid residue of the formula :

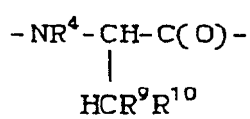

wherein :

$R^4$ is as described hereinabove ;
$R^9$ and $R^{10}$ are independently :
r) hydrogen ;
s) $C_1$-$C_8$ alkyl ;
t) monofluorinated alkyl ;
u) $C_2$-$C_8$ alkenyl ;
v) $C_4$-$C_7$ cycloalkyl ;

or $R^9$ and $R^{10}$ are combined to form a $C_3$-$C_5$ diradical ;

$R^a$ is

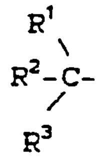 if m is 0; or

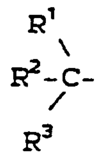 or phenyl if m is 1;

wherein :

$R^1$ is H or $C_1$-$C_6$ alkyl ;

$R^2$ is :

w) $C_3$-$C_7$ cycloalkyl ;

x) $C_5$-$C_7$ cycloalkenyl ;

y) phenyl ;

z) phenyl substituted one to three times by : $-NH_2$. $C_1$-$C_4$ alkyl, $-SR^8$, $-CO_2H$, halogen or $OR^8$ ;

where $R^8$ is H or $C_1$-$C_4$ alkyl ;

aa) polycyclic aromatic ring ;

bb) $C_1$-$C_4$ alkyl monosubstituted by one of the substituents w)-aa) hereinabove, $-CO_2H$ or $-NHR^{11}$ ;

where $R^{11}$ is H or $-CO_2CH_2C_6H_5$ ;

or $R^2$ is H or $C_1$-$C_6$ alkyl if m is 1.

$R^3$ is :

cc) hydrogen ;

dd) phenyl ;

ee) phenyl substituted one to three times by : $-NH_2$,
$C_1$-$C_4$ alkyl, halogen, $-SR^8$, $-CO_2H$, or $OR^8$ ;

where $R^8$ is H, $C_1$-$C_4$ alkyl or phenyl ;

ff) polycyclic aromatic radical ;

gg) heteroaromatic ring system ;

hh) $C_1$-$C_4$ alkyl independently substituted one or two times by the groups dd)-gg) hereinabove or $-CO_2H$ ;

ii) $-NHR^{12}$ ; where $R^{12}$ is hh) hereinabove or hydrogen, $C_1$-$C_6$ alkyl, $-CH_2CO_2H$ or $-C(O)CHR^{13}NH_2$ ;

where $R^{13}$ is H or $C_1$-$C_4$ alkyl monosubstituted by $-SR^8$ or $-CO_2H$.

m is 0 or 1.

n is 1 if m is 0 or n is 2 if m is 1.

Provided that when $A^1$ and $A^2$ are both valine, $R^4$ is hydrogen, $R^5$ is $-NR^6R^7$, $R^6$ is hydrogen, $R^7$ is hydrogen or benzyl, $A^4$ is aspartic acid, $A^5$ is leucine and m is 0, then $R^2$ is not $C_1$-$C_4$ alkyl monosubstituted by one of the substituents w)-aa).

The present invention also provides for the pharmaceutically acceptable salts of the above compounds.

The terms "alkyl, alkenyl and alkynyl" are intended to include linear and branched structures.

The term "alkyl" is intended to include methyl, ethyl, propyl, isopropyl, butyl, sec- and tert-butyl, pentyl, hexyl and the like.

The term "alkenyl" is intended to include vinyl, allyl, isopropenyl, pentenyl, hexenyl and the like.

The term "alkynyl" is intended to include ethynyl, propynyl, butynyl and the like.

The term "cycloalkyl" is intended to include cyclopropyl, cyclobutyl, cyclopentyl, cyclohexyl, cycloheptyl and the like.

The term "cycloalkenyl" is intended to include cyclopentenyl, cyclohexenyl, cycloheptenyl and the like.

The term "heteroaromatic ring system" is intended to include pyridine, thiophene, furan and the like.

The term "heteroaromatic polycyclic ring system" is intended to include quinoline, isoquinoline, indole, benzofuran, benzothiophene and the like.

The term "aromatic polycyclic ring system" is intended to include naphthalene, phenanthrene and the like.

The term "halogen" is intended to include fluorine, chlorine, bromine or iodine atom.

Some of the compounds described herein contain one or more centers of asymmetry and may thus give rise to diastereoisomers and optical isomers. The present invention is meant to comprehend such possible diastereomers as well as their racemic and resolved optically active forms.

Some of the compounds described herein contain olefinic double bonds and unless specified otherwise, are meant to include both E and Z geometric isomers.

Preferably $A^3$ is an amino acid residue having the formula :

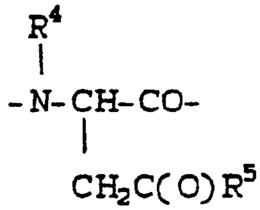

where $R^4$ is hydrogen and $R^5$ is $-NR^6R^7$ where $R^6$ and $R^7$ are independently hydrogen, methyl, ethyl, benzyl, or $R^6$ and $R^7$ are combined to form a $C_3$-$C_5$ diradical or $-NR^6R^7$ is morpholino.

Preferably $A^5$ is an amino acid residue having the formula :

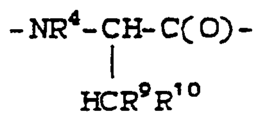

where $R^4$ and $R^9$ are hydrogen, and $R^{10}$ is t-butyl or isopropyl.

Preferably $R^1$ is hydrogen.

Preferably $R^2$ is phenyl, benzyl, or phenyl or benzyl substituted by $-NH_2$, $C_1$-$C_4$ alkyl, $-SR^8$, $CO_2H$ or $OR^8$ ; where $R^8$ is H or $C_1$-$C_4$ alkyl.

One aspect of this invention involves a pharmaceutical composition comprising an antiherpes virally effective amount of a compound of Formula I, or a therapeutically acceptable salt thereof, and a pharmaceutically or veterinarily acceptable carrier.

Another aspect of this invention involves a method of treating herpes viral infection in a mammal by administering to the mammal an antiherpes virally effective amount of the peptide of Formula I or a therapeutically acceptable salt thereof as defined hereinafter.

Another aspect of this invention involves a method of treating viral infections in mammals comprising administering to the mammal an antivirally effective amount of the peptide of Formula I with another antiviral acyclonucleoside or a related compound.

Processes for preparing the peptides of Formula I are described hereinafter.

## DETAILED DESCRIPTION OF THE INVENTION

It has now been found that a series of substituted pentapeptides inhibit the activity of the ribonucleotide reductase enzyme of herpes simplex virus in vitro. This enzyme is required for in vivo replication of the herpes simplex virus. The inhibitory activity has been shown in vitro to be specific for the virus and does not affect mammalian reductase enzymes.

The peptides of the instant invention and amides and salts thereof can be manufactured according to known synthetic methods, i.e., by condensing amino acids stepwise or by solid phase synthesis according to the method originally described by Merrifield, J. Am. Chem. Soc., 85 : 2149-2154 (1963) or by using automated peptide synthesizing equipment. The N-substituent can then be attached to the pentapeptide unit according to known synthetic methods, i.e., by reaction with the corresponding activated acylating agent in the presence of an organic nitrogen base. Examples of "activated acylating agent" may be acid chloride, acid anhydride, pentafluorophenyl ester and carbamyl chloride.

The condensation between two amino acids can be carried out according to the usual condensation methods such as azide method, mixed acid anhydride method, DCC (dicyclohexyl carbodiimide) method, N-hydroxysuccinimide method, cyano method, Woodward reagent K method, carbonyl diimidazole method or oxidation reduction method. These condensation reactions may be done in either liquid or solid phase. In the case of elongating the peptide chain in the solid phase method, the peptide is attached to an insoluble carrier at the C-terminal amino acid. For insoluble carriers, those which react with the carboxy group of the C-terminal amino acid to form a bond which is readily cleaved later, for example, halomethyl resin such as chloromethyl resin and bromomethyl resin, benzhydrylamine resin, and t-alkoxy carbonyl hydrazide resin can be used.

As is usual in peptide synthesis, it is necessary to protect/deprotect the α- and λ-side amino groups and the carboxy group of the amino acid as the occasion demands. The applicable protective groups to amino groups are exemplified such as benzyloxycarbonyl (hereinafter abbreviated as Z), o-chlorobenzyloxy carbonyl [Z(2-C1)], p-nitrobenzyloxy carbonyl [$Z(NO_2)$], p-methoxybenzyloxycarbonyl [Z(-OMe)], t-butoxycarbonyl (BOC), t-amyloxycarbonyl (Aoc), isobornyloxycarbonyl (Bpoc), 9-fluorenylmethoxycarbonyl (Fmoc), methylsulfonyl ethoxy carbonyl (Msc), trifluoroacetyl, phthalyl, formyl, 2-nitrophenyl sulphenyl (NPS), diphenyl phosphinothioyl (Ppt), dimethyl phosphinothioyl (MPT) and the like.

As protective groups for the carboxy group there can be exemplified, for example, benzyl ester (OBzl), 4-nitrobenzyl ester [$OBzl(NO_2)$], t-butyl ester (OBut), 4-pyridyl methyl ester (OPic), and the like. It is desirable that specific amino acids such as arginine, cysteine, and serine possessing a functional groups other than amino and carboxyl groups are protected by a suitable protective group as occasion demands. For example, the guanidino group in arginine may be protected with nitro, p-toluene sulfonyl, benzyloxycarbonyl, adamantyloxycarbonyl, p-methoxybenzenesulfonyl, 4-methoxy-2,3,6-trimethylbenzenesulfonyl (Mtr), 4-methoxy-2,6-dimethyl benzenesulfonyl (Mds), 1,3,5-trimethylphenylsulfonyl (Mts), and the like. The thiol group in cysteine may be protected with benzyl, p-methoxybenzyl, triphenylmethyl, acetylaminomethyl, ethyl carbamoyl, 4-methylbenzyl, 2,4,6-trimethylbenzyl (Tmb), etc, and the hydroxy group in serine can be protected with benzyl, t-butyl, acetyl, tetrahydropyranyl, etc.

Conventional methods of peptide synthesis as described, for example, by Schroder et al., "The Peptides", Vol. I, Academic Press, 1965, or Bodansky et al., "Peptide Synthesis," Interscience Publishers, 1966, or McOmie (ed.), "Protective Groups in Organic Chemistry," Plenum Press, 1973, or Barany et al., "The Peptides: Analysis, Synthesis, Biology," 2 Chapter 1, Academic Press, 1980, the disclosures of which are hereby incorporated by reference.

The pharmaceutical compositions of the present invention comprise a compound of Formula I as an active ingredient or a pharmaceutically acceptable salt, thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary, and tertiary amines, substituted amines including naturally occurring substituted amines, cyclic amines and basic ion exchange resins, such as arginine, betaine, caffeine, choline, $N,N^1$-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are citric, hydrobromic, hydrochloric, maleic, phosphoric, sulfuric and tartaric acids.

In an aspect of the invention there is provided a pharmaceutical composition or preparation comprising a compound of the Formula I as hereinbefore defined ; or a therapeutically acceptable salt thereof, together with a pharmaceutically acceptable carrier therefore. In a particular aspect the pharmaceutical composition comprises a compound of the present invention in effective unit dosage form.

As used herein the term "effective unit dosage" or "effective unit dose" is denoted to mean a predetermined antiviral amount sufficient to be effective against the viral organisms in vivo. Pharmaceutically-acceptable carriers are materials useful for the purpose of administering the medicament, and may be solid, liquid, or gaseous materials, which are otherwise inert and medically acceptable and are compatible with the active ingredients.

These pharmaceutical compositions may be given parenterally, orally, used as a suppository or pessary, applied topically as an ointment, cream, aerosol, powder, or given as eye or nose drops, etc., depending on whether the preparation is used to treat internal or external viral infections.

For internal infections the compositions are administered orally or parenterally at dose levels of about 0.1 to 250 mg per kg, preferably 1.0 to 50 mg per kg of mammal body weight, and are used in man in a unit dosage form, administered, e.g. a few times daily, in the amount of 1 to 250 mg per unit dose.

For oral administration, fine powders or granules may contain diluting, dispersing and/or surface active agents, and may be presented in a draught, in water or in a syrup ; in capsules or sachets in the dry state or in a non-aqueous solution or suspension, wherein suspending agents may be included ; in tablets, wherein binders and lubricants may be included ; or in a suspension in water or a syrup. Where desirable or necessary, flavoring, preserving, suspending, thickening, or emulsifying agents may be included. Tablets and granules are preferred, and these may be coated.

For parenteral administration or for administration as drops, as for eye infections, the compounds may be presented in aqueous solution in a concentration of from about 0.1 to 10%, more preferably 0.1 to 7%, most preferably 0.2% w/v. The solution may contain antioxidants, buffers, etc.

Alternatively, for infections of the eye, or other external tissues, e.g. mouth and skin, the compositions are preferably applied to the infected part of the body of the patient as a topical ointment or cream. The compound may be presented in an ointment, for instance, with a water soluble ointment base, or in a cream, for instance with an oil in a water cream base, in a concentration of from about 0.1 to 10%, preferably 0.1 to 7%, most preferably 1% w/v.

The following examples illustrate the present invention without, however, limiting the same thereto. All temperatures are expressed in degrees Celsius.

EXAMPLE 1

L-Tyrosyl-L-valyl-L-valyl-L-asparagyl-L-aspartyl-L-(γ-methyl)leucine

The title compound was synthesized by using a Milligen 9050 automated peptide synthesizing machine employing the commercially available PepSyn KA resin and the corresponding single peptide units commercially available as the N-9-fluorenylmethoxycarbonyl-pentafluorophenyl esters. The polypeptide-resin product from the synthesizer was treated with 95 : 4 : 1 trifluoroacetic acid : 1,2-ethanedithiol : thioanisole, followed by aqueous 80% acetic acid. The crude product in solution was then purified by reverse phase HPLC to provide the title compound.

p.m.r. ($D_2O$) δ : 0.87-0.98 (4d, 12H), 0.90 (s, 9H), 1.65-1.78 (dq, 2H), 1.93-2.08 (m, 2H), 2.70-2.90 (dq, 4H), 3.11 (d, 2H), 4.03,4.14 (t, 2H), 4.28 (t, 1H), 4.32 (dd, 1H), 6.85,7.81 ppm (q, 4H) ;

M.S. (FAB) : m/e 736 $(M+H)^+$.

By the same method the following compounds were prepared :

L-tyrosyl-L-valyl-L-valyl-L-asparagyl-L-aspartyl-L-(2-allylglycine) ;
L-tyrosyl-L-valyl-L-valyl-L-asparagyl-L-aspartyl-L-isoleucine ;
L-phenylalanyl-L-valyl-L-valyl-L-(O-methyl)aspartyl-L-aspartyl-L-leucine :
L-tyrosyl-L-valyl-L-valyl-L-asparagyl-L-aspartyl-D,L-(α-methyl)leucine ;
L-tyrosyl-L-valyl-L-valyl-L-asparagyl-L-aspartyl-aminocyclopropane carboxylic acid ;
L-tyrosyl-L-leucyl-L-valyl-L-asparagyl-L-aspartyl-L-leucine ;
glycyl-L-tyrosyl-L-valyl-L-valyl-L-(O-methyl-aspartyl)-L-aspartyl-L-leucine ;
and L-tyrosyl-L-(N-methylvalyl)-L-valyl-L-asparagyl-L-aspartyl-L-leucine.

EXAMPLE 2

L-Aspartyl-L-valyl-L-valyl-L-(N′,N′-dimethylasparagyl)-L-aspartyl-L-leucine

Step A : N-Carbobenzyloxyaspartic acid α-Benzyl-γ-p-nitrophenyl diester

A solution of 1.79 g of N-carbobenzyloxy-aspartic acid α-benzyl ester and 835 mg of p-nitrophenol in 10 mL of ethanol was cooled to 0°C and treated with a solution of 1.03 g of dicyclohexylcarbodiimide (DCC) in 3 mL of EtOAc. The mixture was stirred for 45 minutes at 0°C and 2 hours at room temperature (RT). The mixture was filtered and the solid washed with EtOAc. The combined filtrates were concentrated under vacuum and the residue recrystallized from hot EtOH with 1% acetic acid to provide the title compound.

Step B : N-Carbobenzyloxy-N′,N′-dimethylasparagine benzyl ester

To a solution of 613 mg of dimethylamine hydrochloride and 1.36 mL of diisopropylethylamine in 2 ml of methanol and 10 mL of EtOAc at 0°C was added a solution of the diester from Step A (3 g) in EtOAc. The mixture was stirred for 30 minutes at 0°C, then stirred at RT overnight. The solution was then concentrated under vacuum and the residue was flash chromatographed over silica gel (2 : 1 hexane : EtOAc, then 2 : 1 EtOAc :

hexane) to provide the title compound.

Step C : N',N'-Dimethylasparagine

The mixture of 2.1 g of the ester of Step B and 100 mg of 10% Pd on C in 50 mL of acetic acid and 25 mL of $H_2O$ was shaken under a hydrogen atmosphere for 2 hours. The mixture was then filtered through Celite and concentrated under vacuum. The residue was dissolved in methanol and filtered through decolorizing carbon. The filtrate was concentrated under vacuum to provide the title compound.

Step D : N-(Fluorenylmethoxycarbonyl)-N',N'-dimethyl-asparagine

A solution of the asparagine of Step C (441 mg) and 595 mg of trimethylchlorosilane in 10 ml of methylene chloride was refluxed for 1 hour. The mixture was then cooled to 0°C and treated with 1.44 mL of diispropylethylamine. After the mixture became a homogeneous solution 711 mg of 9-fluorenylmethyl chloroformate was added and the solution stirred at 0°C for 20 minutes and RT for 1 hour. The solution was then concentreated under vacuum and the residue was dissolved in a mixture of 40 mL of ethyl ether and 50 mL of 5% aqueous bicarbonate solution. The layers were separated and the aqueous phase was washed 2 times with ethyl ether. The combined organic phases were back extracted with water and the combined aqueous phases were acidified to pH 2. The aqueous mixture was then extracted with ethyl ether (3 × 60 mL) and the combined organic phases were dried over anhydrous sodium sulfate. The solution was then concentrated under vacuum to provide the title compound which was used as is in a subsequent reaction.

Step E : N-(Fluorenylmethoxycarbonyl)-N',N'-dimethyl-asparagine pentafluorophenyl ester

The substituted aparagine of Step D (545 mg) was treated with 263 mg of pentafluorophenol and the mixture dissolved in 10 mL of ethyl acetate. The solution was cooled to 0°C and solution of 295 mg of DCC in 5 mL of ethyl acetate was added. The mixture was stirred for 45 minutes at 0°C and for 75 minutes at RT. The mixture was filtered through a pad of Celite and concentrated under vacuum. Flash chromatography of the residue over silica gel (1 : 1 ethyl acetate : hexane) provided the title compound.

Step F : L-Aspartyl-L-valyl-L-valyl-L-(N',N'-dimethyl-asparagyl)-L-aspartyl-L-leucine

The title compound was prepared using the peptide synthesizing machine procedure of Example 1 but substituting the ester of Step E for the commercially available unsubstituted asparagine.
p.m.r. ($CD_3CO_2D$) δ : 0.85-1.05 (m, 18H), 1.75 (m, 3H), 2.10 (m, 2H), 2.88-3.20 (m, 6H), 2.92 (s, 3H), 3.05 (s, 3H), 4.45 (m, 2H), 4.68 (m, 2H), 5.02 ppm (m, 2H) ;
M.S. (FAB) : m/e 702 $(M+H)^+$.

By the same procedure of Example 2 but substituting the corresponding amine in Step B the following compounds were prepared :

L-tyrosyl-L-valyl-L-valyl-L-(N'-ethyl-N'-methylasparagyl)-L-aspartyl-L-leucine ;
L-tyrosyl-L-valyl-L-valyl-L-(N',N'-dimethylasparagyl)-L-asparatyl-L-leucine ;
L-tyrosyl-L-valyl-L-valyl-L-[N',N'-(oxydiethylene)asparagyl]-L-aspartyl-L-leucine ;
L-aspartyl-L-valyl-L-valyl-L-(N',N'-tetramethyleneasparagyl)-L-aspartyl-L-leucine ;
L-tyrosyl-L-valyl-L-valyl-L-(N',N'-tetramethyleneasparagyl)-L-aspartyl-L-leucine ;
L-tyrosyl-L-valyl-L-valyl-L-(N',N'-dimethylasparagyl)-L-aspartyl-L-(γ-methyl)leucine
L-methionyl-L-tyrosyl-L-valyl-L-valyl-L-(N',N'-dimethylasparagyl)-L-aspartyl-L-(γ-methyl)leucine and
L-tyrosyl-L-valyl-L-valyl-L-(N'-methylasparagyl)-L-aspartyl-L-leucine.

## EXAMPLE 3

D-N-(2-Benzyl-2-(4-methoxybenzyl)acetyl)-L-valyl-L-valyl-L-asparagyl-L-asparatyl-L-leucine and L-N-(2-Benzyl-2-(4-methoxybenzyl)acetyl)-L-valyl-L-valyl-L-asparagyl-L-asparatyl-L-leucine

Step A : 2-Benzyl-2-(4-methoxybenzyl)acetic acid

A solution of 0.673 mL of diisopropylamine and 1.78 mL of a 2.5 M n-butyllithium in hexanes in THF was stirred at 0°C for 1 hour. A solution of 288 mg of 3-(4-methoxyphenyl)propanoic acid in THF was added and the mixture stirred for 1 hour at RT. Hexamethylphosphoramide (0.317 mL) was added and the mixture stirred and additional 10 minutes at RT. The solution was then cooled to 0°C and 0.190 mL of benzyl bromide was added rapidly. The solution was stirred at RT for 1 hour and then cooled to 0°C. IN Aqueous HC1 solution was added to bring the solution to pH 2. The mixture was then extracted 4x with chloroform. The combined organic extracts were washed twice with water and then with brine. The organic phase was then dried over anhydrous sodium sulfate, filtered and concentrated under vacuum. Flash chromatography of the residue over silica gel (50 : 1 methylene chloride : methanol) provided the title compound.

Step B : D,L-2-Benzyl-2-(4-methoxybenzyl)acetic acid pentafluorophenyl ester

Using the procedure of Example 2, Step E but substituting the acid from Example 3, Step A for the asparagine of Example 2, Step E, provided the title compound.

Step C : D,L-N-(2-Benzyl-2-(4-methoxybenzyl)acetyl)- L-valyl-L-valyl-L-asparagyl-L-(O-t-butyl)-aspartyl-L-leucyl-Merrifield resin

A mixture of 433 mg of L-valyl-L-valyl-L- asparagyl-L-(O-t-butyl)aspartyl-L-leucyl-Merrifield resin (produced on the Biosearch 9500 automated peptide synthesizing machine) in 1.5 mL of anhydrous dioxane was swirled for 30 minutes. A solution of the ester of Step B (305 mg) and 0.122 mL of diisopropylethylamine in 1.5 mL of anhydrous dioxane was added and the mixture stirred in a sealed vial on a mechanical stirring shaft for 6 days at RT. At the end of this time the mixture was filtered and the collected solid was washed 3x with 4 mL of ethyl ether, and 3x with methylene chloride. The solid was then dried under vaccum to provide the title compound.

Step D : D-N-(2-Benzyl-2-(4-methoxybenzyl)-acetyl)- L-valyl-L-valyl-L-asparagyl-L-aspartyl- L-leucine and L-N-(2-Benzyl-2-(4-methoxybenzyl)-acetyl)- L-valyl-L-valyl-L-asparagyl-L-aspartyl- L-leucine

A mixture of 440 mg of the resin-bound peptide from Step C, 0.7 mL of anisole and 6 mL of hydrofluoric acid (HF) was stirred 1 hour at RT. The remaining HF was removed under a $N_2$ stream and the residue was stirred in ethyl ether and filtered. The solid was then extracted with 30% aqueous acetic acid and the combined extracts were diluted with water to a concentration of approx. 10% acetic acid. Lyophilization of the solution provided 47 mg of a fluffy solid. HPLC purification of the solid provided two separate isomers of the title compound.

ISOMER A :

p.m.r. ($CD_3CO_2D$) δ : 0.44 (d, 3H), 0.65 (d, 3H), 0.85-1.05 (m, 12H), 1.35 (m, 1H), 1.60-1.82 (m, 4H), 2.68-3.08 (m, 9H), 3.79 (s, 3H), 4.22 (m, 1H), 4.30 (m, 1H), 4.65 (m, 1H), 4.96 (t, 2H), 6.79 (d, 2H), 7.06 (d, 2H), 7.15 (m, 1H), 7.75 ppm (m, 4H) ;

M.S. (FAB) : m/e 811 $(M+H)^+$.

ISOMER B :

p.m.r. ($D_2O$) δ : 0.45 (d, 3H), 0.63 (d, 3H), 0.90-1.05 (m, 12H), 1.60-2.10 (m, 5H), 2.73-3.06 (m, 9H), 3.73 (s, 3H), 4.19 (d, 1H), 4.24 (d, 1H), 4.63 (m, 1H), 4.95 (t, 2H), 6.80 (d, 2H), 7.17 ppm (m, 7H) ;

M.S. (FAB) : m/e 811 $(M+H)^+$.

EXAMPLE 4

L-[N-(2,2-Dibenzylacetyl)valyl]-L-valyl-L-aspargyl-L-aspartyl-L-leucine

Using the procedure of Example 3, Step B and C, but substituting dibenzylacetic acid for the acid of Example 3, Step A, provided the title compound.

p.m.r. ($D_2O$) δ : 0.37 (d, 3H), 0.60 (d, 3H), 0.86 (m, 12H), 1.59 (m, 3H), 2.0 (m, 1H), 2.53-3.0 (m, 8H), 3.06 (m, 1H), 3.71 (d, 1H), 3.88 (d, 1H), 4.18 (m, 1H), 4.61 (m, 1H), 4.72 (m, 1H), 7.44 (m, 10H), 7.80 (d, 1H), 7.92 ppm (d, 1H) ;

M.S. (FAB) : m/e 781 $(M+H)^+$.

Using this procedure but substituting the corresponding acid for dibenzylacetic acid, the following compounds were synthesized :

L-[N-(3,3-diphenylpropanoyl)valyl]-L-valyl-L-asparagyl-L-aspartyl-L-leucine ;
L-[N-(2-benzylacetyl)valyl]-L-valyl-L-asparagyl-L-aspartyl-L-leucine ;
L-[N-(2,2-diphenylacetyl)valyl]-L-valyl-L-asparagyl-L-aspartyl-L-leucine.

EXAMPLE 5

L-[N-(2,2-Dibenzylacetyl)valyl]-L-valyl-L-(N',N'-dimethylasparagyl)-L-aspartyl-L-(γ-methyl)leucine

Using the procedure of Example 4, but substituting L-valyl-L-valyl-L-(N',N'-dimethyl-asparagyl-L-aspartyl-L-leucyl PepSyn KA resin for the Merrifield resin bound peptide in Example 4 provided the title compound. p.m.r. ($CD_3CO_2D$) δ : 0.85-1.05 (m, 21H), 1.63 (m, 1H), 1.85 (broad s, 2H), 2.05 (m, 2H), 2.70-3.10 (m, 15H), 4.20 (m, 2H), 4.62 (dd, 1H), 4.98 (m, 2H), 7.18 ppm (m, 10H) ;
M.S. (FAB) : m/e 823 $(M+H)^+$.

EXAMPLE 6

N-(p-Hydroxyhydrocinnamyl)-L-valyl-L-valyl-L-asparagyl-L-aspartyl-L-leucine

Step A : t-Butyl p-t-butoxyhydrocinnamate

A solution of 10 g of p-hydroxyhydrocinnamic acid in 100 mL of dioxane was cooled to 0°C and treated with 10 mL of concentrated sulfuric acid. Isobutylene (50 mL) was then added to the reaction and the mixture shaken for 3 hours at RT. The remaining isobutylene was removed, and the solution was then poured into a saturated solution of sodium bicarbonate. The aqueous mixture was extracted 4x with EtOAc and the combined organic extracts were dried over magnesium sulfate, filtered and concentrated under vacuum. The crude oil was dissolved in ether and the solution was washed 6x with 1 N aqueous NaOH solution. The organic solution was then dried over magnesium sulfate, filtered and concentrated under vacuum. Chromatography of the residue over silica gel (9 : 1 hexane : EtOAc) provided the title compound.

Step B : p-t-Butoxyhydrocinnamic acid

A solution of 968 mg of the ester from Step A in 16 mL of methanol was treated with a solution of 600 mg of potassium hydroxide in 20 mL of water. The mixture was refluxed overnight then cooled to RT and filtered. The filtrate was concentrated under vacuum. The residue was dissolved in water and the solution cooled to 0°C. A 2N aqueous HC1 solution was added to bring the pH to 5 and the resulting white solid was collected and washed 4x with water. The solid was dried overnight under vacuum over $P_2O_5$ to provide the title compound.

Step C : p-t-Butoxyhydrocinnamic acid pentafluorophenyl ester

Using the procedure for Example 2, Step E, but substituting the acid of Step B for the derivatized aparagine of Example 2, provided the title compound.

Step D : N-(p-Hydroxyhydrocinnamyl)-L-valyl-L-valyl-L-asparagyl-L-aspartyl-L-leucine

Using the procedure of Example 1 but substituting p-t-butoxyhdyrocinnamic acid pentafluorophenyl ester for the final single peptide unit charged into the peptide synthesizer provided the title compound.
p.m.r. ($CD_3CO_2D$) δ : 0.84-0.95 (m, 12H), 1.68 (m, 2H), 2.56 (m, 2H), 2.75-3.00 (m, 4H), 4.30 (m, 2H), 4.54 (m, 1H), 4.91 (m, 2H), 6.73-7.00 (q, 4H), 7.78,7.80, 7.99,8.14,8.22 ppm (d, 5H) ;
M.S. (FAB) : m/e 707 $(M+H)^+$.

EXAMPLE 7

N-[2,2-Bis(4-hydroxybenzyl)acetyl]-L-valyl-L-valyl-L-asparagyl-L-aspartyl-L-leucine

Step A : 4-(t-Butyldimethylsiloxy)benzoic acid

A solution of 8.3 g of 4-hydroxybenzoic acid, 15.0 g of t-butyldimethylsilyl chloride, and 25 g of imidazole in 200 mL of THF and 30 mL of dimethylformamide (DMF) was stirred overnight at RT. The solution was then concentrated under vacuum and the residue was dissolved in pH 7 buffer and water. The aqueous solution was extracted 2x with EtOAc and was then acidified with 3N aqueous hydrochloric acid (HC1) solution. The aqueous mixture was then extracted 3x with EtOAc and the extracts were combined and washed 2x with water and once with brine. The organic solution was dried over anhydrous sodium sulfate, filtered and concentrated under vacuum. The residue was dissolved in 50 mL of methanol (MeOH) and 8 mL of triethylamine. The solution was stirred for 2 hours at RT and water was added. The methanol was removed under vacuum and the aqueous residue was acidified to pH 1.5 with 2 N aqueous HC1 solution. The aqueous mixture was extracted 4x with EtOAc and the combined extracts washed with water and brine. The organic phase was dried over sodium sulfate, filtered and concentrated under vacuum to provide the title compound.

Step B : 4-(t-Butyldimethylsiloxy)benzyl alcohol

The acid from Step A (10 g) was dissolved in 80 mL of THF and the solution cooled to 0°C. A 1 M lithium aluminum hydride (LAH) in THF solution (53 ml) was added dropwise and the mixture stirred for 2 hours at RT. A saturated aqueous sodium sulfate solution (4 ml) was added dropwise and a small volume of THF was added to facilitate stirring. The mixture was stirred overnight at RT, then filtered through a pad of Celite. The cake of precipitate was redissolved and stirred in hot THF and this mixture filtered. The hot THF treatment was repeated twice and all of the filtrates, including the initial filtrate, were combined and concentrated under vacuum. The residue was distilled under reduced pressure to provide the title compound (bp : 138° (2 mm)).

Step C : 4-(t-Butyldimethylsiloxy)benzyl chloride

A solution of 6.68 g of the alcohol from Step B and 9.12 g of triphenylphosphine in 140 mL of carbon tetrachloride was refluxed at 110°C for 24 hours. The mixture was filtered and the filtrate concentrated under vacuum. The residue was taken up in hexane and the mixture filtered. The precipitate was washed with hexane until no more product remained in the solid. The filtrate was then concentrated under vacuum and the residue was distilled under reduced pressure to provide a cloudy liquid. Flash chromatography of the liquid over silica gel (100 : 1 hexane : EtOAc) provided a clear liquid that was azeotroped 3x with toluene. The reside was then filtered through a millipore acrodisc to provide the title compound.

Step D : p-(t-Butyldimethylsiloxy)hydrocinnamic acid

Using the procedure of Step A but substituting p-hydroxyhydrocinnamic acid for 4-hydroxybenzoic acid provided the title compound as a crystalline solid. mp 84.5-85.5°C.

Step E : 2,2-Bis(p-(t-butyldimethylsiloxy)benzyl)-acetic acid

A solution of 0.673 mL of diisopropylamine in 3.0 mL of anhydrous THF was cooled to 0°C and the solution treated with 1.72 mL of a 2.4 M n-butyl-lithium in hexane solution. The solution was stirred at 0°C for 45 minutes and then a solution of 448.5 mg of the acid from Step D in 2.5 mL of anhydrous THF was added, followed by 0.317 mL of HMPA. The solution was stirred for 1 hour at RT, then the solution was cooled to-20°C and 410 mg of p-(t-butyldimethylsiloxy)benzyl chloride was added. The solution was stirred for 1.5 hours at RT then cooled to 0°C. A 2 N aqueous HC1 solution was added until pH 2 was achieved and the mixture was extracted 4x with EtOAc. The combined organic phases were washed 3x with water and once with brine. The organic solution was dried over anhydrous sodium sulfate, then filtered and concentrated under vacuum. Chromatography of the residue over silica gel (gradient 2.5 : 1 to 1 : 2 hexane : EtOAc) provided the title compound as a glassy paste.

Step F : 2,2-Bis(p-(t-butyldimethylsiloxy)benzyl)-acetic acid pentafluorophenyl ester

Using the procedure of Example 2, Step E, but substituting the acid from Step E for the acid of Example 2, Step D, provided the title compound.

Step G : N-[2,2-Bis(4-hydroxybenzyl)acetyl]-L-valyl-L-valyl-L-asparagyl-L-aspartyl-L-leucine

Using the procedure Example 3, Steps C and D, but substituting the ester from Step F for the ester of Example 3, Step B, provided the title compound.
p.m.r. ($CD_3OD$) δ : 0.63 (d, 3H), 0.75 (d, 3H), 0.9-1.05 (m, 12H), 1.65 (m, 2H), 1.80 (m, 3H), 2.70 (m, 4H), 2.88 (m, 5H), 3.99 (d, 1H), 4.05 (d, 1H), 4.48 (broad, 1H), 4.70 (m, 1H), 4.80 (m, 1H), 6.70 (m, 4H), 7.04 ppm (m, 4H) ;
M.S. (FAB) : m/e 813 $(M+H)^+$.

EXAMPLE 8

N-(2,2-Dibenzylacetyl)-L-valyl-L-valyl-L-asparagyl-L-aspartyl-L-(γ-methylleucine)

Step A : 2,2-Dibenzylacetic anhydride

A solution of 41 mg of DCC in 0.1 mL of methylene chloride was added to a solution of dibenzylacetic acid in 0.4 mL of methylene chloride at 0°C and the mixture stirred for 10 minutes at 0°C. The slurry was then filtered and the solid washed twice with methylene chloride. The combined filtrates were concentrated under vacuum to provide the title compound as a colorless glass.

Step B : N-(2,2-Dibenzylacetyl)-L-valyl-L-valyl-L-asparagyl-L-(O-t-butyl)aspartyl-L-(γ-methylleucyl) PepSyn KA resin

A solution of 96 mg of the anhdyride from Step A and 6.1 mg of 4-(dimethylamino)pyridine (DMAP) in 2 mL of DMF was added to dry L-valyl-L-valyl-L-asparagyl-L-(O-t-butyl)aspartyl-L-(γ-methylleucyl) pepSyn KA resin (synthesized using a Milligen 9050 automated peptide synthesizing machine) and the mixture left at RT for 24 hours, swirled occasionally in the sealed flask. The mixture was then filtered and the solid was washed 4x with anhdyrous DMF, 3x with methylene chloride and 3x with ether. The solid was dried under vacuum to provide the title compound as a colorless powder.

Step C : N-(2,2-Dibenzylacetyl)-L-valyl-L-valyl-L-asparagyl-L-aspartyl-L-(γ-methylleucine)

The resin bound peptide of Step B was suspended in 4 mL of a 95 : 4 : 1 trifluoroacetic acid : ethanedithiol: thioanisole and the mixture gently stirred for 2 hours. The mixture was then filtered and the solid stirred with 4 mL of the same solution for 5 minutes. The mixture was again filtered and the solid then extracted with 4 mL of 80% aqueous acid. The combined extracts were concentrated under vacuum and the residue was extracted 4x with ether. HPLC purification of the insoluble material provided the title compound.
p.m.r. ($D_2O$) δ : 0.35 (d, 3H), 0.59 (d, 3H), 0.91 (d, 3H), 0.95 (d, 3H), 0.92 (s, 9H), 1.57 (m, 1H), 1.99 (m, 1H), 2.51-3.09 (m, 9H), 3.70 (d, 1H), 3.89 (d, 1H), 4.18 (dd, 1H), 4.62 (dd, 1H), 4.72 (dd, 1H),7.20-7.36 ppm (m, 10H) ;
M.S. (FAB) : m/e 795 $(M+H)^+$.

EXAMPLE 9

N-(2,2-Dibenzylacetyl)-L-valyl-L-valyl-L-(N'-benzyl-asparagyl)-L-aspartyl-L-(γ-methylleucine)

Step A : N'-Benzylasparagine

A solution of 5.0 g of aspartic acid β-methyl ester hydrochloride and 8.98 mL of benzylamine in 40 mL of MeOH was stirred at 64°C for 4.5 hours. A colorless crystalline solid began to form after 30 minutes. The mixture was then cooled to RT and filtered. The solid was washed 5x with MeOH and 2x with ether. The solid was then dried under vacuum to provide the title compound as a colorless crystalline powder ; m.p. : 257-260°C.

Step B : N'-(9-Fluorenylmethoxycarbonyl)-N'-benzyl-asparagine

A mixture of 3.64 g of the benzylasparagine from Step A and 4.15 mL of trimethylsilylchloride in 40 mL of methylene chloride was stirred at a gentle reflux for 1 hour. The mixture was then cooled to 0°C and 8.57 mL of diisopropylethylamine was added slowly. 9-Fluorenylmethyl chloroformate (4.04 g) was added and the mixture stirred 20 minutes at 0°C and 1 hour at RT. The clear solution was then concentrated under vacuum to provide a viscous gum which was partitioned between 110 mL of 2.5% aqueous sodium bicarbonate and 100 mL of ether. The phases were separated and the thick aqueous phase was washed 3x with ether. The aqueous phase was then carefully acidified to pH 2 by the slow addition of 2N aqueous HC1. The mixture was then filtered and the solid dried under vacuum to provide the title compound as a colorless powder ; m.p. : 176-178°C.

Step C : N'-(9-Fluorenylmethoxycarbonyl)-N'-benzyl-asparagine pentafluorophenyl ester

A solution of 822 mg of DCC in 5 mL of EtOAc was added to a slurry of 1.99 g of the substituted asparagine of Step B and 787 mg of pentafluorophenol in 5 mL of EtOAc at 0°C. The mixture was stirred for 1 hour at 0°C and 1 hour at RT. The mixture was then diluted with 40 mL of methylene chloride and the mixture then filtered. The solid was washed with methylene chloride and 2x with ether. The solid was then dried under vacuum and the solid then extracted 2x with THF and 2x with ether. The remaining solid was dried and then extracted 2x with THF and 2x with ether. The combined extracts were concentrated under vacuum to provide a mixture of the title compound and dicyclohexylurea in a 5.3 : 1 ratio. This mixture was employed without further purification.

Step D : L-Valyl-L-valyl-L-(N′-benzylasparagyl)-L-aspartyl-L-(γ-methylleucyl) PepSyn KA resin

The title compound was synthesized using a Milligen 9050 automated peptide synthesizing machine employing the commercially available resin, the corresponding single peptide units commercially available as N-9-fluorenylmethoxycarbonyl-pentafluorophenyl esters and the ester from Step C.

Step E : N-(2,2-Dibenzylacetyl)-L-valyl-L-valyl-L-(N′-benzylasparagyl)-L-aspartyl-L-(γ-methyl-leucine)

Using the procedure of Example 8, Step C, but substituting the resin bound peptide of Step B for the resin bound protein of Example 8, Step B, provided the title compound.
p.m.r. ($D_2O$) δ : 0.33 (d, 3H), 0.55 (d, 3H), 0.88 (d, 3H), 0.90 (s, 9H), 0.91 (d, 3H), 1.56, 1.69 (dq, 2H), 1.55 (m, 1H), 1.96 (m, 1H), 2.50-3.08 (m, 9H), 3.71 (d, 1H), 3.90 (d, 1H), 4.16 (dd, 1H), 4.34 (s, 2H), 4.62 (dd, 1H), 4.74 (dd,1H), 7.20-7.42 ppm (m, 15H) ;
M.S. (FAB) : m/e 885 $(M+H)^+$.

EXAMPLE 10

L-Tyrosyl-L-valyl-L-valyl-L-(O-methylaspartyl)-L-aspartyl-L-(γ-methylleucine)

Using the procedure of Example 9, Step B thru E, but substituting aspartic acid β-methyl ester for the substituted asparagine of Example 9, Step A, provided the title compound.
p.m.r. ($D_2O$) δ : 0.87-0.95 (m, 12H), 0.92 (s, 9H), 1.65, 1.77 (dq, 2H), 2.00 (m, 2H), 2.72-2.98 (m, 4H), 3.70 (s, 3H), 4.01 (d, 1H), 4.15 (d, 1H), 4.28 (m, 2H), 6.85,7.10 ppm (q, 4H) ;
M.S. (FAB) : m/e 751 $(M+H)^+$.

EXAMPLE 11

N-(9-Fluorenylmethoxycarbonyl)-D-α-methylphenylalanyl-L-valyl-L-valyl-L-asparagyl-L-aspartyl-L-leucine and N-(9-Fluorenylmethoxycarbonyl)-L-α-methylphenylalanyl-L-valyl-L-valyl-L-asparagyl-L-aspartyl-L-leucine

Step A : D,L-N-(9-Fluorenylmethoxycarbonyl)-α-methylphenylalanine pentafluorophenyl ester

Using the procedure of Example 9, Steps B and C, but substituting α-methylphenylalanine for the substituted asparagine of Example 9, Step A, provided the title compound.

Step B :
N-(9-Fluorenylmethoxycarbonyl)-D-α-methylphenylalanyl-L-valyl-L-valyl-L-asparagyl-L-aspartyl-L-leucine and N-(9-Fluorenylmethoxycarbonyl)-L-α-methylphenylalanyl-L-valyl-L-valyl-L-asparagyl-L-aspartyl-L-leucine

Using the procedure of Example 3, Steps C and D, but substituting the ester of Step A for the ester of Example 3, Step B, provided a crude mixture of the two diastereomers of the title compound. A portion of the mixture was separated into the individual pure diastereomers in the HPLC purification, while another portion was employed as a mixture in the synthesis of Example 12.
ISOMER A
p.m.r. ($D_2O$) δ : 1.02 (m, 18H), 1.25 (b, 3H), 1.70 (m, 3H), 2.15 (m, 1H), 2.80 (m, 4H), 3.15 (m, 2H), 4.18 (d, 1H), 4.20 (d, 1H), 4.28 (t, 1H), 4.42 (b, 2H), 4.71-4.79 (m, 3H), 7.0-8.0 ppm (m, 13H) ;
M.S. (FAB) : m/e 942 $(M+H)^+$.
ISOMER B :
p.m.r. ($D_2O$) δ : 1.02 (m, 18H), 1.28 (b, 3H), 1.70 (m, 3H), 2.18 (m, 1H), 2.80 (m, 4H), 3.10 (m, 2H), 4.17 (d, 1H), 4.20 (d, 1H), 4.28 (t, 1H), 4.42 (b, 2H), 4.80-4.90 (m, 3H), 6.9-8.1 ppm (m, 13H) ;
M.S. (FAB) : m/e 942 $(M+H)^+$.

EXAMPLE 12

D-α-Methylphenylalanyl-L-valyl-L-valyl-L-asparagyl-L-aspartyl-L-leucine and L-α-Methylphenylalanyl-L-valyl-L-valyl-L-asparagyl-L-aspartyl-L-leucine

The mixture of the isomers of the substituted peptide of Example 11 (70 mg) was dissolved in 1 mL of dimethylformamide (DMF) and 0.111 mL of diethylamine was added to the solution. The solution was stirred for 2 hours at RT then concentrated under vacuum. The residue was triturated with ether and then with hexanes. HPLC purification of the residue provided two isomers of the title compound.

ISOMER A :

p.m.r. ($D_2O$) δ : 0.83 (d, 3H), 0.89 (d, 3H), 1.00 (m, 12H), 1.49 (s, 3H), 2.09 (m, 2H), 2.80 (m, 5H), 3.22 (d, 1H), 4.00 (d, 1H), 4.25 (m, 2H), 4.72 (m, 1H), 4.85 (m, 1H), 7.35 ppm (m, 5H) ;

M.S. (FAB) : m/e 767 $(M+H)^+$.

ISOMER B :

p.m.r. ($D_2O$) δ : 0.98 (m, 12H), 1.04 (d, 3H), 1.06 (d, 3H), 1.48 (s, 3H), 2.09 (m, 2H), 2.76 (m, 5H), 3.25 (d, 1H), 4.14 (d, 1H), 4.23 (m, 1H), 4.69 (m, 1H), 4.82 (m, 1H), 7.32 ppm (m, 5H) ;

M.S. (FAB) : m/e 767 $(M+H)^+$.

EXAMPLE 13

D,L-(α-Methyltyrosyl)-L-valyl-L-valyl-L-asparagyl-L-aspartyl-L-leucine

Step A : D,L-N-(9-Fluorenylmethoxycarbonyl)-α-methyltyrosine pentafluorophenyl ester

Using the procedure of Example 9, Steps B and C, but substituting α-methyltyrosine for the substituted asparagine of Example 9, Step A, provided the title compound.

Step B :
D,L-[N-(9-Fluorenylmethyoxycarbonyl)-α-methyltyrosyl]-L-valyl-L-valyl-L-asparagyl-L-aspartyl-L-leucine

Using the procedure of Example 3, Steps C and D, but substituting the ester of Step A for the ester of Example 3, Step B, provided the title compound.

Step C : D,L-(α-Methyltyrosyl)-L-valyl-L-valyl-L-asparagyl-L-aspartyl-L-leucine

Using the procedure of Example 12 but substituting the peptide of Step B for the peptide of Example 11 and piperidine for diethylamine provided two isomers of the title compound which were separated by the HPLC purification.

ISOMER A

p.m.r. ($D_2O$) δ : 0.98 (m, 18H), 1.72 (m, 3H), 1.85 (s, 3H), 1.93 (m, 1H), 2.92 (m, 4H), 3.17 (d, 1H), 3.30 (d, 1H), 4.44 (m, 2H), 4.58 (m,1H), 4.95 (m, 2H), 6.80-7.02 (q, 4H), 7.82 (d, 1H), 8.20 (d, 1H), 8.34 (d, 1H), 8.39 (d, 1H), 8.40 ppm (d, 1H) ;

M.S. (FAB) : m/e 736 $(M+H)^+$.

ISOMER B :

p.m.r. ($D_2O$) δ : 0.98 (m, 18H), 1.71 (m, 3H), 1.74 (s, 3H), 1.88 (m, 1H), 2.92 (m, 4H), 3.28 (d, 1H), 3.34 (d, 1H), 4.28-4.38 (m, 2H), 4.57 (m, 1H), 4.95 (m, 1H), 6.86-7.12 (q, 4H), 7.82 (d, 1H), 8.10 (d, 1H), 8.13 (d, 1H), 8.19 (d, 1H), 8.30 ppm (d, 1H) ;

M.S. (FAB) : m/e 736 $(M+H)^+$.

EXAMPLE 14

L-[N-(4-Biphenylmethyl)tyrosyl]-L-valyl-L-valyl-L-asparagyl-L-aspartyl-L-leucine

A 1N aqueous sodium hydroxide (NaOH) solution (0.055 mL) was added to a slurry of 20.0 mg of L-tyrosyl-L-valyl-L-valyl-L-asparagyl-L-aspartyl-L-leucine (synthesized using a Milligen 9050 automated peptide synthesizing machine) in 0.165 mL of water. The cloudy solution was diluted with 0.22 mL of acetonitrile and then a solution of 20.2 mg of biphenyl-4-carboxaldehyde in 0.2 mL of acetonitrile was added. The mixture was stirred for 1 hour at RT and then a solution of 4.2 mg of sodium borohydride in 0.2 mL of acetonitrile and 0.05 mL of water was added. The mixture was stirred for 1.5 hours at RT and then 0.2 mL of acetic acid was added. The fine colorless suspension was concentrated under vacuum and the residue was washed 4x with ethyl ether. HPLC purification of the residue provided the title compound as colorless powder.
p.m.r. ($D_2O$) δ : 0.81-1.01 (m, 18H), 1.60 (m, 2H), 1.88 (m, 1H), 2.04 (m, 1H), 2.60-3.22 (3xdq, 6H), 4.00 (d, 1H), 4.09 (d, 1H), 4.19 (m, 2H), 6.83,7.08 (q, 4H), 7.48-7.80 ppm (m, 9H) ;
M.S. (FAB) : m/e 888 $(M+H)^+$.

By the same method the following compounds were prepared by substituting the corresponding aldehyde for biphenyl-4-carboxaldehyde and the corresponding peptide for the peptide employed in Example 14 :

L-[N-benzyltyrosyl]-L-valyl-L-valyl-L-asparagyl-L-aspartyl-L-leucine ;
L-[N-(3-phenoxybenzyl)tyrosyl]-L-valyl-L-valyl-L-asparagyl-L-aspartyl-L-leucine ;
L-(N-benzyltyrosyl)-L-valyl-L-valyl-L-asparagyl-L-aspartyl-L-(γ-methylleucine) ;
L-[N-(3-thienylmethyl)tyrosyl]-L-valyl-L-valyl-L-asparagyl-L-aspartyl-L-leucine ;
L-[N-(carboxymethyl)tyrosyl]-L-valyl-L-valyl-L-asparagyl-L-aspartyl-L-leucine and
L-[N-(4-carboxybenzyl)tyrosyl]-L-valyl-L-valyl-L-asparagyl-L-aspartyl-L-leucine.

## EXAMPLE 15

### L-Tyrosyl-L-valyl-L-valyl-L-asparagyl-L-aspartyl-L-(N-methylleucine)

#### Step A : N-(9-Fluorenylmethoxycarbonyl)-N-methyl-L-leucine

Using the procedure of Example 9, Step B, but substituting N-methyl-L-leucine for the substituted asparagine of Example 9, Step A, provided the title compound.

#### Step B : N-(9-Fluorenylmethoxycarbonyl)-N-methyl-L-leucyl anhydride

Using the procedure of Example 8, Step A, but substituting the substituted leucine of Step A for dibenzylacetic acid provided the title compound.

#### Step C : N-(9-Fluorenylmethoxycarbonyl)-N-methyl-L-leucyl PepSyn KA resin

A solution of 143 mg of the leucyl anhydride of Step B in 2 mL of DMF was added to 0.5 g of the PepSyn KA resin. A solution of 6.1 mg of 4-N,N-dimethylaminopyridine in 0.5 mL of DMF was added and the mixture was gently shaken. The mixture was let stand for 24 hours, with occasional stirring. The mixture was then filtered and the solid washed 4x with DMF, 3x with methylene chloride and 3x with ether. Drying under vacuum provided the title compound as a colorless powder.

#### Step D : L-Tyrosyl-L-valyl-L-valyl-L-asparagyl-L-aspartyl-L-(N-methylleucine)

Using the procedure of Example 1 but substituting the peptide unit of Step C for the commercially available γ-methylleucine unit provided the title compound.
p.m.r. ($D_2O$) δ : 0.81-0.99 (m, 15H), 1.38 (m, 1H) 1.62-1.83 (m, 2H), 1.98 (m, 2H), 2.50-2.93 (m, 4H), 3.04 (s, 3H), 3.12 (d, 2H), 4.04 (d, 1H), 4.15 (d, 1H), 4.27 (t,1H), 4.94 (dd, 1H), 5.27 (dd, 1H), 6.85 7.10 ppm (q, 4H) ;
M.S. (FAB) : m/e 736 $(M+H)^+$.

## EXAMPLE 16

### L-[N-(Dibenzylcarbamyl)valyl]-L-valyl-L-asparagyl-L-aspartyl-L-leucine

#### Step A : Dibenzylcarbamyl chloride

Pyridine (1.75 g) and 8 mL of benzene were added to a mixture of 4.4 g of dibenzylamine and 12 mL of toluene and the mixture cooled to 0°C. A 20% phosgene in toluene solution (15 mL) was added over a 45 minute period. The pasty mixture was diluted with 10 mL of toluene and stirred gently at RT for 18 hours. The mixture was filtered and the filtrate concentrated under vacuum. Chromatography of the residue over silica gel (2 : 1 hexane : EtOAc) provided the title compound.

Step B : L-[N-(Dibenzylcarbamyl)valyl]-L-valyl-L-asparagyl-L-aspartyl-L-leucyl-PepSyn KA resin

Using the procedure of Example 8, Steps C and D, but substituting the chloroformamide from Step A for the ester of Example 8, Step B, and PepSyn KA resinbound peptide for the corresponding Merrifield resin bound peptide employed in Example 8, provided the title compound.

Step C : L-[N-Dibenzylcarbamyl)valyl]-L-valyl-L-asparagyl-L-aspartyl-L-leucine

Using the procedure of Example 8, Step C, but substituting the resin bound peptide of Step B for the resin bound peptide of Example 8. Step C, provided the title compound.
p.m.r. ($CD_3OD$) δ : 0.77 (d, 3H), 0.88 (d, 3H), 0.95 (d, 3H), 1.0 (m, 9H), 1.65 (m, 1H), 1.80 (m, 2H), 2.04 (m, 1H), 2.14 (m, 1H), 2.70 (dd, 1H), 2.80-294 (m, 3H), 4.17 (t, 2H), 4.40-4.55 (m, 3H), 4.66-4.76 (m, 3H), 4.80 (t, 1H) 7.30 (m, 6H), 7.36 (m, 4H), 7.98-8.10 ppm (m, 1H) ;
M.S. (FAB) : m/e 782 $(M+H)^+$.

EXAMPLE 17

L-[N-(Diphenylcarbamyl)valyl]-L-valyl-L-asparagyl-L-aspartyl-L-leucine

Using the procedure of Example 3, Step C, but substituting diphenylcarbamyl chloride for the ester of Example 3 Step B, and DMF for dioxane, provided the title compound.
p.m.r. ($CD_3CO_2D$) δ : 0.84-1.05 (m, 18H), 1.66-1.82 (m, 3H), 1.96-2.14 (m, 2H), 2.82-3.06 (m, 4H), 4.27 (d, 1H), 4.44 (d, 1H), 4.63 (m, 1H), 4.98 (m, 2H), 7.30 (m, 6H), 7.40 ppm (m, 4H) ;
M.S. (FAB) : m/e 754 $(M+H)^+$.

EXAMPLE 18

L-(N-Benzyltyrosyl)-L-valyl-L-valyl-L-(N′,N′-dimethyl-asparagyl)-L-aspartyl-L-(γ-methylleucine)

Step A : N-(Fluorenylmethoxycarbonyl)-N-benzyl-L-(4-t-butoxyphenylalanine)

Using the procedure of Example 2, Step D, but substituting N-benzyl-(4-t-butoxyphenylalanine) for the asparagine of Example 2, Step C, provided the title compound.

Step B : L-(N-Benzyltyrosyl)-L-valyl-L-valyl-L-(N′,N′-dimethylasparagyl)-L-aspartyl-L-(γ-methylleucine

Using the procedure of Example 8, Steps A through C, but substituting the derivatized phenylalanine of Step A for dibenzylacetic acid and the corresponding resin-bound pentapeptide for the resin-bound pentapeptide on Example 8, Step B, provided the title compound.
p.m.r. ($D_2O$) δ : 0.87-0.99 (m, 12H), 0.93 (s, 9H), 1.65, 1.73 (dq, 2H), 1.92 (m, 1H), 2.03 (m, 1H), 2.55-3.30 (m, 6H), 2.91 (s, 3H), 3.20 (s, 3H), 3.55 (dd, 1H), 3.63, 3.79 (q, 2H), 3.98 (d, 1H), 4.07 (d, 1H), 4.19 (dd, 1H), 4.65 (dd, 1H), 6.80, 7.04 (q, 4H), 7.30-7.46 ppm (m, 5H) ;
M.S. (FAB) : m/e 854 $(M+H)^+$.

EXAMPLE 19

L-[N-Bis(1-naphthylmethyl)acetylvalyl]-L-valyl-L-asparagyl-L-aspartyl-L-leucine

Step A : Dimethyl-2,2-bis(1-naphthylmethyl)malonate

A 2.0 N methanolic sodium hydroxide solution (62 mL) was added to a solution of 14.2 mL of dimethylmalonate in 50 mL of methanol was added. The mixture was stirred 30 mins. at RT and then a solution of 25 g of 1-bromomethylnaphthalene in 50 mL of methanol was added. The mixture was heated at 50°C for 1 hour, then filtered and concentrated under vacuum. The residue was dissolved in 200 mL of EtOAc and the solution was washed 4x with 1N aqueous HC1 solution, then with brine. The organic phase was dried over magnesium sulfate, then filtered and concentrated under vacuum. Chromatography of the residue over silica gel (5 : 1 hexane : EtOAc) provided the title compound along with dimethyl-2-(1-naphthylmethyl)malonate.

Step B : 2,2-Bis(1-naphthylmethyl)acetic acid

A solution of 2.74 g of the bis(naphthylmethyl)malonate in 10 mL of methanol was treated with 27 mL of 1 N aqueous sodium hydroxide. The mixture was heated at reflux (105°C) overnight. The mixture was then cooled to RT and then diluted with 70 mL of water. The aqueous solution was washed 2x with EtOAc and the aqueous phase then cooled to 0°C. The aqueous solution was slowly acidified to pH 1 with 6N aqueous HC1 solution. The mixture was then extracted 5x with ether. The combined ether extracts were washed with brine, dried over magnesium sulfate, filtered and concentrated under vacuum to provide the title compound.

Step C : 2,2-Bis(1-naphthylmethyl)acetyl chloride

A suspension of 230 mg of the acid of Step B in 4 mL of toluene was treated with 1 mL of oxalyl chloride and the reaction mixture stirred for 45 minutes at RT. The solution was then concentration under vacuum to provide the title compound.

Step D : L-[N-Bis(1-naphthylmethyl)acetylvalyl]-L-valyl-L-asparagyl-L-aspartyl-L-leucine

A suspension of L-valyl-L-valyl-L-asparagyl-L-aspartyl-L-leucine (prepared using Biosearch 9500 automated peptide synthesizing machine and cleaving the resin as described in Example 3, Step D) and 0.108 mL of trimethylsilyl chloride in methylene chloride was stirred at RT for 3 hours. Diisopropylethylamine (0.186 mL) was added, then 76.8 mg of the acid chloride from Step C was added and the reaction stirred at RT for 3 hours. The reaction mixture was then concentrated under vacuum and the residue dissolved in ether and aqueous sodium bicarbonate solution. The aqueous phase was washed with ether and then acidified. The aqueous mixture was then extracted 3x with EtOAc. The combined organic extracts were dried over magnesium sulfate, filtered and evaporated. HPLC purification of the residue provided the title compound.
p.m.r. ($D_2O$) δ : 0.28 (d, 3H), 0.52 (d,3H), 0.90 (m, 12H), 2.53 (m, 1H), 2.62 (m, 2H), 2.75-2.84 (m, 4H), 4.05 (m, 1H), 4.15 (m, 1H), 4.50 (m, 1H),4.85 (m, 2H), 7.32-7.72 ppm (m, 7H) ;
M.S. (FAB) : m/e 881 $(M+H)^+$.

EXAMPLE 20

L-(3,5-Diiodotyrosyl)-L-valyl-L-valyl-L-asparagyl-L-aspartyl-L-leucine

Step A : L-(3,5-Diiodotyrosyl)-L-valyl-L-valyl-L-asparagyl-L-(O-t-butyl)aspartyl-L-leucyl PepSyn KA resin

A solution of 15.8 mg of 4-methylmorpholine, 684 mg of (N-fluorenylmethoxycarbonyl)-3,5-diiodotyrosine, 46.2 mg of [benzotriazolyloxy]tris[dimethylamino]phosphonium hexafluorophosphate, and 16.0 mg of 1-hydroxybenzotriazole monohydrate in 0.75 mL of DMF was stirred for 10 minutes, then 300 mg of L-valyl-L-valyl-L-asparagyl-L-(O-t-butyl)aspartyl-L-leucyl PepSyn KA resin (synthesized using a Milligen 9050 automated peptide synthesizing machine) was added. The mixture was diluted with 0.9 mL of DMF and swirled 24 hours at RT. The mixture was then treated with 2 mL of 20% v/v piperidine/DMF solution and the mixture swirled for 15 minutes. The liquid was decanted and the solid treated with 3 mL of the piperidine/DMF solution and swirled for 15 minutes. The liquid was again decanted and the solid washed 4x with DMF and 3x with methylene chloride to provide the title compound.

Step B : L-(3,5-Diiodotyrosyl)-L-valyl-L-valyl-L-asparagyl-L-aspartyl-L-leucine

Using the procedure of Example 8, Step C, but substituting the resin bound peptide of Step A for the resin bound peptide of Example 8, Step C, provided the title compound.
p.m.r. ($CD_3OD$) δ : 0.90-1.10 (m, 18H), 1.65 (m, 1H), 1.80 (m, 2H), 2.15 (m, 2H), 2.75 (m, 2H), 2.88 (m, 3H), 3.20 (dd, 1H), 4.14-4.32 (m, 3H), 4.44 (m, 1H), 4.74 (t, 1H), 4.81 (t, 1H), 7.76 (s, 2H), 8.08-8.31 ppm (m, 3H);
M.S. (FAB) : m/e 973 $(M+H)^+$.

Using the same procedure as steps A and B above, but substituting L-valyl-L-valyl-L-(N',N'-dimethylasparagyl)-L-(O-t-butyl)aspartyl-L-(γ-methylleucyl)-PepSyn KA resin for the resin bound peptide above, provided L-(3,5-diiodotyrosyl)-L-valyl-L-valyl-L-(N'N'-dimethylasparagyl)-L-aspartyl-L-(γ-methylleucine).
p.m.r. ($CD_3OD$) δ : 1.00 (m, 27H), 1.80-1.99 (m, 2H), 2.05-2.22 (m, 2H), 2.79-2.96 (m, 4H), 3.05 (m, 1H), 3.21 (dd, 1H), 4.16 (dd, 1H), 4.23 (m, 1H), 4.40 (d, 1H), 4.46 (m, 1H), 4.80 (m, 2H), 7.75 (s, 2H), 8.20 ppm (t, 2H) ;
M.S. (FAB) : m/e 1016 $(M+H)^+$.

EXAMPLE 21

L-[N'-Benzyloxycarbonyllysyl]-L-valyl-L-valyl-L-asparagyl-L-aspartyl-L-leucine

Using the procedure of Example 20, but substituting N-(fluorenylmethoxycarbonyl)-N'-benzyl-oxycarbonyllysine for the derivatized tyrosine employed in Example 20, provided the title compound.
p.m.r ($CD_3OD$) δ : 1.00 (m, 18H), 1.40-2.20 (m, 11H), 2.75 (m, 1H), 2.89 (m, 2H), 3.05 (m, 1H), 3.16 (m, 2H), 4.02 (m, 1H), 4.27 (m, 2H), 4.45 (m, 1H), 4.74 (m, 1H), 4.82 (broad t, 1H), 5.11 (s, 2H), 7.38 (m, 5H), 7.70-8.35 ppm (m, 4H) ;
M.S. (FAB) : m/e 821 $(M+H)^+$.

EXAMPLE 22

L-Lysyl-L-valyl-L-valyl-L-asparagyl-L-aspartyl-L-leucine

Using the procedure of Example 2, Step C, but substituting the hexapeptide of Example 21 for the dimethylasparagine of Example 2, Step B, provided a crude residue which was purified by HPLC to provide the title compound.
p.m.r. ($CD_3OD$) δ : 1.00 (m, 18H), 1.55 (m, 2H), 1.75 (m, 5H), 1.95 (m, 2H), 2.15 (m, 2H), 2.75 (m, 2H), 2.86 (m, 4H), 3.02 (t, 2H), 4.03 (t, 1H), 4.18 (m, 1H), 4.26 (m, 1H), 4.44 (m, 1H), 4.71 (m, 1H), 4.81 (t, 1H), 8.18-8.34 ppm (m, 2H) ;
M.S. (FAB) : m/e 687 $(M+H)^+$.

EXAMPLE 23

L-Tyrosyl-L-valyl-L-valyl-L-asparagyl-L-aspartyl-L-(5-fluoronorvaline)

Step A : 5-Fluoronorvaline

L-Norvaline (11.72 g) was placed in a large quartz reactor vessel and the vessel cooled to -80°C and 100 ml of anhydrous hydrofluoric acid was condensed inside. The vessel was irradiated with UV light and trifluoromethylhypofluorite was bubbled into the vessel for 5 hours. A total of 80 psig of the hypofluorite was used. The UV irradiation was continued for an additional 45 minutes, then the solution was stored overnight at -80°C. The reaction solution was then concentrated under a stream of nitrogen and the residue was then dissolved in 2 N aqueous HC1 soltuion. This aqueous solution was concentrated under vacuum. HPLC purification of the residue provided the hydrochloride salt of the title compound as well as 4-fluoronorvaline hydrochloride.

The hydrochloride salt of 5-fluoronorvaline was dissolved in 10 mL of water and the solution filtered thru a Celite plug. The solution was then diluted with isopropyl alcohol (iPrOH) to 75 mL volume and 2 mL of propylene oxide was added. The solution was stirred until the free amino acid began to crystallize out and then it was allowed to stand for 30 minutes. The mixture was diluted with 50 mL of iPrOH and stored in the refrigerator overnight. The mixture was then filtered and the crystals washed with iPrOH and ether. The crystals were then dried under vacuum to provide the title compound.

Step B : L-Tyrosyl-L-valyl-L-valyl-L-asparagyl-L-aspartyl-L-(5-fluoronorvaline)

Using the procedure of Example 15, but substituting the 5-fluoronorvaline of Step A for N-methyl-L-leucine, provided the title compound.
p.m.r. ($D_2O$) δ : 0.89-1.00 (4xd, 12H), 1.70-1.90 (m, 4H), 2.00 (m, 2H), 2.71-2.96 (dq, 4H), 3.11 (d, 2H), 4.05 (d,1H), 4.15 (d, 1H), 4.28, 4.46 (dt, 2H) 4.40 (dd, 1H), 4.71-4.80 (m, 2H), 6.83, 7.10 ppm (q, 4H) ;
M.S. (FAB) m/e 726 $(M+H)^+$.

EXAMPLE 24

L-Tyrosyl-L-valyl-L-valyl-L-asparagyl-L-aspartyl-L-norvaline

L-Tyrosyl-L-valyl-L-valyl-L-asparagyl-L-aspartyl-L-(2-allylglycine) from Example 1 was dissolved in 2 mL of 10% aqueous acetic acid and 13.5 mg of 10% Pd on C was added. The mixture was stirred under a 1 atm. pressure hydrogen atmosphere for 2 hours at RT. The reaction mixture was then filtered and the the solid was washed with 10% aqueous AcOH, 80% aqueous AcOH and 50% aqueous AcOH. The combined filtrates were lyophilized. HPLC purification of the residue provided the title compound.
p.m.r. ($D_2O$) δ : 0.87-1.00 (m, 15H), 1.31 (m, 2H), 1.71 (m, 2H), 2.01 (m, 2H), 2.63-2.87 (dq, 4H), 3.11 (d, 2H), 4.04 (d, 1H), 4.14 (m, 2H), 4.27 (t, 1H), 4.64 (dd, 1H), 4.73 (dd, 1H), 6.85, 7.10 ppm (q, 4H) ;
M.S. (FAB) m/e 708 $(M+H)^+$.

## Claims

1. A peptide having the Formula I :

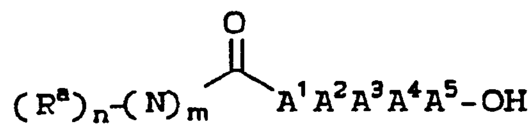

I

wherein :
$A^1$ and $A^2$ are independently
a) isoleucine ;
b) leucine ;
c) norleucine ;
d) valine ;
e) cyclohexylglycine ;
f) phenylglycine ;
g) N-methyl isoleucine ;
h) N-methyl valine ;
i) phenylalanine ;
j) N-methyl leucine ;
or any of the enantiomorphic forms thereof ;
$A^3$ is

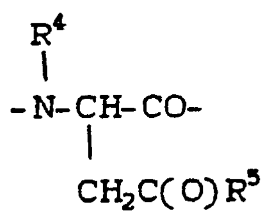

wherein :
$R^4$ is hydrogen or methyl ;
$R^5$ is $-NR^6R^7$ or $-OR^8$
where $R^6$ and $R^7$ are independently :
j) hydrogen ;
k) $C_3$-$C_7$ cycloalkyl ;
l) $C_5$-$C_7$ cycloalkenyl ;
m) phenyl ;
n) phenyl substituted by : $-NH_2$, $C_1$-$C_4$alkyl, $-SR^8$, $-CO_2H$ or $OR^8$ ; where $R^8$ is H or $C_1$-$C_4$ alkyl ;
o) polycyclic aromatic ring system ;
p) $C_1$-$C_6$ alkyl ;
q) $C_1$-$C_4$ alkyl monosubstituted by k)-n) hereinabove
or $R^6$ and $R^7$ are combined to form a $C_3$-$C_6$ diradical or $-NR^6R^7$ is morpholino ;
$A^4$ is aspartic acid, N-methylaspartic acid, or any of the enantiomorphic forms thereof ;
$A^5$ is an amino acid residue of the formula :

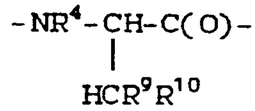

wherein :
$R^4$ is as described hereinabove ;
$R^9$ and $R^{10}$ are independently :
r) hydrogen ;
s) $C_1$-$C_6$ alkyl ;
t) monofluorinated alkyl ;
u) $C_2$-$C_6$ alkenyl ;
v) $C_4$-$C_7$ cycloalkyl ;
or $R^9$ and $R^{10}$ are combined to form a $C_3$-$C_6$ diradical ;
$R^a$ is

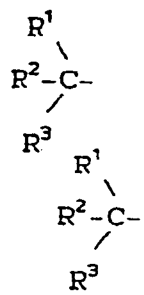

if m is 0; or

or phenyl if m is 1;

wherein :
$R^1$ is hydrogen or $C_1$-$C_6$ alkyl ;
$R^2$ is :
w) $C_3$-$C_7$ cycloalkyl ;
x) $C_5$-$C_7$ cycloalkenyl ;
y) phenyl ;
z) phenyl substituted one to three times by : $-NH_2$, $C_1$-$C_4$ alkyl, $-SR^8$, $-CO_2H$, halogen, or $-OR^8$ ;
where $R^8$ is H or $C_1$-$C_4$ alkyl ;
aa) polycyclic aromatic ring system ;
bb) $C_1$-$C_4$ alkyl monosubstituted by w)-aa) hereinabove, $-CO_2H$ or $-NHR^{11}$ ;
where $R^{11}$ is H or $-CO_2CH_2C_6H_5$ ;
or $R^2$ is hydrogen or $C_1$-$C_6$ alkyl if m is 1 ;
$R^3$ is ;
cc) hydrogen ;
dd) phenyl ;
ee) phenyl substituted one to three times by : $-NH_2$, $C_1$-$C_4$ alkyl, $-SR^8$, $-CO_2H$, halogen or $-OR^8$ ;
where $R^8$ is H, phenyl or $C_1$-$C_4$ alkyl ;
ff) polycyclic aromatic ring system ;
gg) heteroaromatic ring system ;
hh) $C_1$-$C_4$ alkyl independently substituted one or two times by the groups dd)-gg) hereinabove or $-CO_2H$;
ii) $-NHR^{12}$ ;
where $R^{12}$ is hh) hereinabove, hydrogen, $-CH_2CO_2H$, $-C(O)CHR^{13}NH_2$ or $C_1$-$C_6$ alkyl ; where $R^{13}$ is H or $C_1$-$C_4$ alkyl monosubstituted by $-SR^8$ or $-CO_2H$ ;
m is 0 or 1 ;
n is 1 if m is 0 or n is 2 if m is 1 ;
provided that when $A^1$ and $A^2$ are both valine, $R^4$ is hydrogen, $R^5$ is $-NR^6R^7$, $R^6$ is hydrogen, $R^7$ is hydrogen or benzyl, $A^4$ is aspartic acid, $A^5$ is leucine and m is 0, then $R^2$ is not $C_1$-$C_4$ alkyl monosubstituted by one of the substituents w)-aa) ;

and the pharmaceutically acceptable salts thereof.

2. A peptide of Formula I of Claim 1 wherein :
$A^1$ and $A^2$ are independently :
a) isoleucine ;
b) leucine ;
c) norleucine ;
d) valine ;
e) cyclohexylglycine ;
f) phenylglycine ;
g) N-methyl isoleucine ;
h) N-methyl valine ;
i) phenylalanine ;
j) N-methyl leucine ;
or any of the enantiomorphic forms thereof ;
$A^3$ is an amino acid residue having the formula :

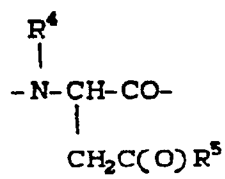

where $R^4$ is hydrogen and $R^5$ is $-NR^6R^7$ ;
where $R^6$ and $R^7$ are independently hydrogen, methyl, ethyl, benzyl, or $R^6$ and $R^7$ are combined to form a C3-C5 diradical or $-NR^6R^7$ is morpholino. ;
$A_4$ is aspartic acid, N-methylaspartic acid, or any of the enantiomorphic forms thereof ;
$A^5$ is a peptide having the formula :

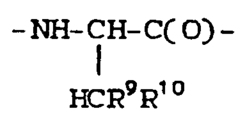

where $R^9$ is hydrogen and $R^{10}$ is isopropyl or t-butyl ;
$R^1$ is hydrogen ;
$R^2$ is phenyl, benzyl or phenyl or benzyl substituted by $-NH_2$, $C_1$-$C_4$ alkyl, $-SR^8$, $-CO_2H$, or $-OR^8$ ;
where $R^8$ is hydrogen or $C_1$-$C_4$ alkyl ;
m is 0 and n is 1
provided that when $A^1$ and $A^2$ are both valine, $R^4$ is hydrogen, $R^5$ is $-NR^6R^7$, $R^6$ is hydrogen, $R^7$ is hydrogen or benzyl, $A^4$ is aspartic acid and $A^5$ is leucine, then $R^2$ is not $C_1$-$C_4$ alkyl monosubstituted by one of the substitutents w)-aa) ;
and the pharmaceutically acceptable salts thereof.

3. A method for the synthesis of a peptide of Formula I :

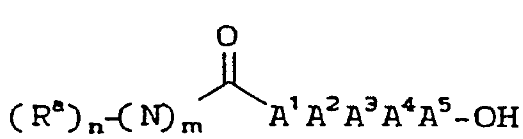

I

Ra is

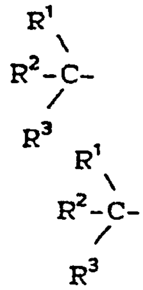

if m is 0; or

or phenyl if m is 1;

wherein :
$R^1$ is hydrogen or $C_1$-$C_6$ alkyl ;
$R^2$ is :

w) $C_3$-$C_7$ cycloalkyl ;
x) $C_5$-$C_7$ cycloalkenyl ;
y) phenyl ;
z) phenyl substituted one to three times by : $-NH_2$, $C_1$-$C_4$ alkyl, $-SR^8$, $-CO_2H$, halogen, or $-OR^8$ ;
where $R^8$ is H or $C_1$-$C_4$ alkyl ;
aa) polycyclic aromatic ring system ;
bb) $C_1$-$C_4$ alkyl monosubstituted by w)-aa) hereinabove, $-CO_2H$ or $-NHR^{11}$ ;
where $R^{11}$ is H or $-CO_2CH_2C_6H_5$ ;

or $R^2$ is hydrogen or $C_1$-$C_6$ alkyl if m is 1 ;
$R^3$ is ;

cc) hydrogen ;
dd) phenyl ;
ee) phenyl substituted one to three times by : $-NH_2$, $C_1$-$C_4$ alkyl, $-SR^8$, $-CO_2H$, halogen or $-OR^8$ ;
where $R^8$ is H, phenyl or $C_1$-$C_4$ alkyl ;
ff) polycyclic aromatic ring system ;
gg) heteroaromatic ring system ;
hh) $C_1$-$C_4$ alkyl independently substituted one or two times by the groups dd)-gg) hereinabove or $-CO_2H$;

ii) $-NHR^{12}$ ;
where $R^{12}$ is hh) hereinabove, hydrogen, $-CH_2CO_2H$, $-C(O)CHR^{13}NH_2$ or $C_1$-$C_6$ alkyl ; where $R^{13}$ is H or $C_1$-$C_4$ alkyl monosubstituted by $-SR^8$ or $-CO_2H$ ;

m is 0 or 1 ;
n is 1 if m is 0 or n is 2 if m is 1 ;
provided that when $A^1$ and $A^2$ are both valine, $R^4$ is hydrogen, $R^5$ is $-NR^6R^7$, $R^6$ is hydrogen, $R^7$ is hydrogen or benzyl, $A^4$ is aspartic acid, $A^5$ is leucine and m is 0, then $R^2$ is not $C_1$-$C_4$ alkyl monosubstituted by one of the substiuents w)-aa) ;
and the pharmaceutically acceptable salts thereof ;
which comprises the reaction of a resin-bound pentapeptide of the Formula II :

$A_1A_2A_3A_4A_5$ PepSyn KA resin II

wherein $A_1$,$A_2$,$A_3$,$A_4$ and $A_5$ are as described hereinabove ; with a substituted activated acylating agent of Formula III or Formula IV :

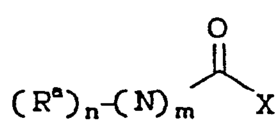

III

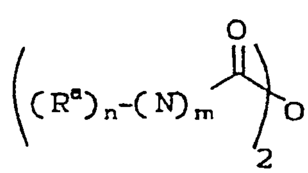

IV

wherein $R^a$, m and n are described hereinabove and X is a halogen or pentafluorophenoxy ; in the presence of an organic nitrogen base and subsequently removing the resin to yield the product.

4. An antiviral pharmaceutical composition comprising an effective amount of a compound of Claim 1 and a pharmaceutically acceptable carrier.

5. The use of a compound of Claim 1 for the manufacture of a medicament for treating virus infections in mammals.

6. The use of a compound of Claim 1 in combination with another antiviral acyclonucleoside or related compound for the manufacture of a medicament for treating virus infections in mammals.

European Patent Office

# EUROPEAN SEARCH REPORT

Application Number

EP 90 31 3148

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 292 255 (MERCK & CO.)<br>* Entire document *<br>--- | 1-6 | C 07 K 7/06<br>A 61 K 37/64 |
| X | PEPTIDES, CHEMISTRY AND BIOLOGY, PROCEEDINGS OF THE TENTH AMERICAN PEPTIDE SYMPOSIUM, St. Louis, Missouri, 23rd - 28th May 1987, pages 638-640; P. GAUDREAU et al.: "Synthesis and biological activity of peptides inhibiting herpes simplex virus ribonucleotide reductase"<br>* Entire document *<br>--- | 1-6 | |
| D,X | THE JOURNAL OF BIOLOGICAL CHEMISTRY, vol. 262, no. 26, 15th September 1987, pages 12413-12416, The American Chemical Society for Biochemistry and Molecular Biology, Inc., US; P. GAUDREAU et al.: "Structure-activity studies on synthetic peptides inhibiting herpes simplex virus ribonucleotide reductase"<br>* Entire document *<br>----- | 1-6 | |
| | | | TECHNICAL FIELDS SEARCHED (Int. Cl.5) |
| | | | C 07 K<br>A 61 K |

The present search report has been drawn up for all claims

| Place of search | Date of completion of the search | Examiner |
|---|---|---|
| THE HAGUE | 08-03-1991 | GROENENDIJK M.S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)